# EUROPÄISCHE PATENTSCHRIFT

(11) **EP 0 680 377 B1**
(45) Veröffentlichungstag und Bekanntmachung des Hinweises auf die Patenterteilung: **02.04.1997**
(21) Anmeldenummer: 94905049.6
(22) Anmeldetag: 13.01.1994
(51) Int. Cl.: B01J 23/00, C07C 29/154

(54) **KATALYSATOREN MIT HOCHDISPERSER VERTEILUNG DER AKTIVKOMPONENTE**
CATALYSTS WITH HIGHLY DISPERSED ACTIVE COMPONENTS
CATALYSEURS A REPARTITION FORTEMENT DISPERSEE DES COMPOSANTS ACTIFS

(30) Priorität: 21.01.1993 DE 4301469
(43) Veröffentlichungstag der Anmeldung: 08.11.1995
(73) Patentinhaber: BASF AKTIENGESELLSCHAFT, 67056 Ludwigshafen (DE)
(72) Erfinder: FETZER, Thomas, D-67346 Speyer (DE); BUECHELE, Wolfgang, D-67063 Ludwigshafen (DE); IRGANG, Matthias, D-69121 Heidelberg (DE); OTTO, Bernhard, D-67117 Limburgerhof (DE); WISTUBA, Hermann, D-68259 Mannheim (DE); BUERGER, Gert, D-68199 Mannheim (DE)
(86) Internationale Anmeldenummer: EP9400083
(87) Internationale Veröffentlichungsnummer: WO9416815

(56) Entgegenhaltungen:
- EP-A- 0 034 338
- EP-A- 0 123 233
- EP-A- 0 348 263
- EP-A- 0 382 164

## Beschreibung

Die Erfindung betrifft Katalysatoren mit hochdisperser Verteilung der Aktivkomponente sowie ein Verfahren zur Herstellung dieser Katalysatoren.

WO-A-89/00082 und die darin aufgeführten Literaturzitate beschreiben die Herstellung von oxidischen Katalysatoren, die Cu, Zn und Al enthalten, wobei als Precursor eine Cu-Zn-Al-Legierung eingesetzt wird, die über einen Oxidationsvorgang teilweise oder vollständig in das Oxid überführt wird. Die Katalysatoren werden zur Herstellung von Methanol eingesetzt.

Aus der DE-A-37 17 111 ist ein Verfahren zur Herstellung eines Kupfer enthaltenden Katalysators zur Tieftemperaturkonvertierung, der neben Kupferoxid noch Zinkoxid und Aluminiumoxid enthält, bekannt. Der Katalysator wird durch Fällung der wasserlöslichen Salze aus wäßriger Lösung mit einem alkalischen Fällungsmittel hergestellt.

Die bekannten Katalysatoren lassen bezüglich Standzeit bzw. Aktivität zu wünschen übrig.

Der vorliegenden Erfindung lag daher die Aufgabe zugrunde, den zuvor genannten Nachteilen abzuhelfen.

Demgemäß wurden neue und verbesserte Katalysatoren, hergestellt durch Vereinigung von M-Al₂O₄, wobei M für ein Element der I., VII. und VIII. Nebengruppe des Periodensystems der Elemente steht, mit Zinn, Blei, einem Element der II. Haupt- oder Nebengruppe des Periodensystems der Elemente als Oxid oder Salz oder in elementarer Form und Calcinieren bei Temperaturen von 300 bis 1300°C und Drücken von 0,1 bis 200 bar gefunden sowie das Verfahren zu ihrer Herstellung.

Die erfindungsgemäßen Katalysatoren können wie folgt hergestellt werden:

Man kann von einem oxidischen Festkörper, der teilweise oder vollständig - also zu 1 bis 100 Gew.-%, bevorzugt 10 bis 90 Gew.-%, besonders bevorzugt 20 bis 70 Gew.-% - ein Spinell der Zusammensetzung M-Al₂O₄ in einer Al₂O₃-Matrix ist, ausgehen und diesen mit gleicher oder höherer Konzentration von Zinn, Blei, einem Element der II. Haupt- oder Nebengruppe des Periodensystems der Elemente als Oxid oder Salz oder in elementarer Form vermischen und bei Temperaturen von 300 bis 1300°C, bevorzugt 500 bis 1200°C, besonders bevorzugt 600 bis 1100°C und Drücken von 0,1 bis 200 bar, bevorzugt 0,5 bis 10 bar, besonders bevorzugt bei Normaldruck (Atmosphärendruck) calcinieren.

Das Vermischen kann z.B. durch Versprühen, mechanisches Vermischen, Verrühren und Verkneten des gemahlenen Festkörpers der Zusammensetzung M-Al₂O₄, bevorzugt in Al₂O₃, besonders bevorzugt in γ-Al₂O₃ oder bevorzugt durch Imprägnieren eines ungemahlenen Festkörpers der Zusammensetzung M-Al₂O₄, bevorzugt in Al₂O₃, besonders bevorzugt in γ-Al₂O₃ mit einer Lösung von Salzen von Zinn, Blei, einem Element der II. Haupt- oder Nebengruppe des Periodensystems der Elemente erfolgen.

Die Freisetzung des Elementes M in elementarer oder oxidischer Form, das in der Regel zur hochdispersen Verteilung führt, kann dadurch induziert werden, daß durch den Calcinierungsschritt die Elemente Zinn, Blei, der II. Haupt- oder Nebengruppe des Periodensystems der Elemente in elementarer, oxidischer oder salzartiger Form das Element M im Spinell substituiert, wenn der entstehende Spinell thermodynamisch stabiler ist als der Ausgangsspinell M-Al₂O₄.

Als Metall M in den Ausgangsoxiden M-Al₂O₄ eignen sich Elemente der I., VII. und VIII. Nebengruppe des Periodensystems der Elemente in der Oxidationsstufe +2 wie Mn²⁺, Fe²⁺, Co²⁺, Ni²⁺ und Cu²⁺, insbesondere Ni²⁺ und Cu²⁺ oder deren Gemische.

Als Elemente der II. Haupt- oder Nebengruppe des Periodensystems der Elemente eignen sich das Element (Metall), oxidische oder salzartige Verbindungen. Als salzartige Verbindungen seien beispielsweise Carbonate, Hydroxide, Carboxylate, Halogenide und oxidische Anionen wie Nitrite, Nitrate, Sulfite, Sulfate, Phosphite, Phosphate, Pyrophosphate, Halogenite, Halogenate und basische Carbonate, bevorzugt Carbonate, Hydroxide, Carboxylate, Nitrate, Nitrite, Sulfate, Phosphate und basische Carbonate, besonders bevorzugt Carbonate, Hydroxide, basische Carbonate und Nitrate, bevorzugt in der Oxidationsstufe +2 wie Zn²⁺, Mg²⁺, Ca²⁺, Sr²⁺ und Ba²⁺, insbesondere Zn²⁺ und Mg²⁺ oder deren Gemische genannt.

Die Herstellung des Ausgangsoxides der Zusammensetzung M-Al₂O₄, bevorzugt in Form eines Spinelles ist Z.B. aus WO-A-82/00820, SU-A-426 968, FR-A-2 655 878 und Rev. Chim. 20 (2) (1969), 105 bis 106 bekannt. Vorteilhaft erweist sich die Tränkung eines Al₂O₃-Trägers mit einer löslichen Verbindung wie einem Salz des Kations M, z.B. einem Nitrit, Nitrat, Sulfit, Sulfat, Carbonat, Hydroxid, Carboxylat, Halogenid, Halogenit und Halogenat und anschließender thermischer Zersetzung des Anions zum Oxid. Eine weitere Möglichkeit ist das Mischen einer Verbindung wie einem Salz des Kations R mit einer sauerstoffhaltigen Aluminiumverbindung, z.B. durch Trocken oder in Suspension, insbesondere durch Sprühtrocknung, Verdichtung des Materials, z.B. durch Verkneten, gegebenenfalls durch Zugabe eines geeigneten Verformungshilfsmittels, Formgebung durch Extrudieren, Trocknung und anschließender Calcinierung zur Bildung des Spinells. Die Calciniertemperatur kann zwischen 300 und 1300°C, bevorzugt zwischen 600 und 1000°C, liegen.

Die Dotierung von hochoberflächigen Aluminiumoxidträgern, d.h. die Bildung von Mischoxiden, führt zur Erhöhung der thermischen Beständigkeit des Trägers (z.B. DE-A-34 03 328, DE-A-25 00 548, Appl. Catal. 7, 211 bis 220 (1983), J. Catal. 127, 595 bis 604 (1991)). Zusätzlich können die Fremdionen zur katalytischen Aktivität des Katalysators beitragen. Zur Dotierung können im allgemeinen folgende Elemente herangezogen werden: Alkalimetalle, Metalle der seltenen Erden, Sc, Ti, V, Cr, Y, Zr, B, Si, Ge, P, Bi. Der Substitutionsgrad von Aluminiumoxid kann z.B. bei 0,01 bis 20 Gew.-% liegen.

Die Partikelgröße des Metalloxides des Elements M im ungebrauchten Katalysator liegt zwischen 1 und 200 nm, bevorzugt zwischen 3 und 100 nm, besonders bevorzugt zwischen 10 und 50 nm. Die Bestimmung der Partikelgröße kann z.B. durch XRD (X-ray defraction) oder TEM (Transmissionselektronenmikroskopie) erfolgen.

Die erfindungsgemäßen Katalysatoren enthalten Mesoporen von 2 bis 20 nm und Makroporen von mehr als 20 nm und BET-Oberflächen zwischen 1 und 350 m²/g, bevorzugt zwischen 10 und 200 m²/g, besonders bevorzugt zwischen 25 und 150 m²/g und die Porosität zwischen 0,01 und 0,8 ml/g.

Die erfindungsgemäßen Katalysatoren eignen sich beispielsweise zur CO-Konvertierung und zur Methanolsynthese.

### Beispiele

### Herstellung des Katalysators

### Beispiel 1

Eine Mischung aus 284 g Puralox® SCF (Fa. Condea), 166 g Pural® SB (Fa. Condea) und 100 g CuO (Fa. Merck) wurde mit 20 ml Ameisensäure (gelöst in 140 ml H₂O) 0,75 h verknetet, zu 3 mm Strängen extrudiert, getrocknet und 4 h bei 800°C calciniert.

Man erhielt einen CuAl₂O₄-haltigen Festkörper mit einer BET-Oberfläche von 112 m²/g und einer bimodalen Porenradienverteilung mit 25 % der Poren im Bereich von 10 - 1 Mikrometer Porendurchmesser und 65 % der Poren im Bereich von 20 - 5 Nanometer Porendurchmesser.

71,4 g des oben beschriebenen CuAl₂O₄-haltigen Festkörpers (Wasseraufnahme: 69,1 %) wurden zweimal mit 49 ml einer salpetersauren (pH 3) wäßrigen Lösung, die 32,6 g Zn(NO₃)₂ enthält, imprägniert und eine Stunde bei Raumtemperatur belassen. Der imprägnierte Träger wurde bis zur Gewichtskonstanz bei 120°C getrocknet und abschließend 4 h bei 600°C calciniert.

Man erhielt einen ZnAl₂O₄-haltigen Festkörper unter Bildung von CuO mit einer BET-Oberfläche von 82 m²/g und einer unveränderten bimodalen Porenradienverteilung.

Die Kristallitgröße des Kupferoxids wurde röntgenographisch bestimmt und beträgt 28 nm.

## Patentansprüche

1. Katalysatoren hergestellt durch Vereinigung von M-Al₂O₄, wobei M für ein Element der I., VII. und VIII. Nebengruppe des Periodensystems der Elemente steht, mit Zinn, Blei, einem Element der II. Haupt- oder Nebengruppe des Periodensystems der Elemente als Oxid oder Salz oder in elementarer Form, und Calcinieren bei Temperaturen von 300 bis 1300°C und Drücken von 0,1 bis 200 bar.

2. Verfahren zur Herstellung von Katalysatoren, dadurch gekennzeichnet, daß man Oxide der Formel M-Al₂O₄, in der M für ein Element der I., VII. und VIII. Nebengruppe des Periodensystems der Elemente steht, mit Zinn, Blei, einem Element der II. Haupt- oder Nebengruppe des Periodensystems der Elemente als Oxid oder Salz oder in elementarer Form vereinigt und bei Temperaturen von 300 bis 1300°C und Drücken von 0,1 bis 200 bar calciniert.

3. Verfahren zur Herstellung von Katalysatoren nach Anspruch 2, dadurch gekennzeichnet, daß der Substituent M in den Oxiden M-Al₂O₄ für ein Element der I., VII. und VIII. Nebengruppe des Periodensystems der Elemente steht.

4. Verfahren zur Herstellung von Katalysatoren nach Anspruch 2, dadurch gekennzeichnet, daß man Zink, Magnesium, Calcium, Strontium oder Barium als Oxid oder Salz oder in elementarer Form verwendet.

5. Verfahren zur Herstellung von Katalysatoren nach Anspruch 2, dadurch gekennzeichnet, daß die Verbindungen der I., VII. und VIII. Nebengruppe des Periodensystems der Elemente Oxide von Mangan, Eisen, Cobalt, Nickel oder Kupfer sind.

## Claims

1. A catalyst prepared by combining M-Al₂O₄ where M is an element of Group Ib, VIIb or VIII of the Periodic Table of the Elements with tin, lead, an element of group IIa or IIb of the Periodic Table of the Elements as oxide or salt or in elemental form and calcining at 300-1300°C under 0.1-200 bar.

2. A process for preparing catalysts, which comprises combining oxides of the formula M-Al₂O₄ where M is an element of Group Ib, VIIb or VIII of the Periodic Table of the Elements with tin, lead, an element of group IIa or IIb of the Periodic Table of the Elements as oxide or salt or in elemental form and calcining at 300-1300°C under 0.1-200 bar.

3. A process for preparing catalysts as claimed in claim 2, wherein the substituent M in the oxides M-Al₂O₄ is an element of Group Ib, VIIb or VIII of the Periodic Table of the Elements.

4. A process for preparing catalysts as claimed in claim 2, wherein zinc, magnesium, calcium, strontium or barium is used as oxide or salt or in elemental form.

5. A process for preparing catalysts as claimed in claim 2, wherein the compounds of the elements of Group Ib, VIIb and VIII of the Periodic Table of the Elements are oxides of manganese, iron, cobalt, nickel or copper.

## Revendications

1. Catalyseurs préparés par la réunion de M-Al₂O₄, où M représente un élément des premier, septième et huitième sous-groupes du système périodique des éléments, avec l'étain, le plomb, un élément du second groupe principal ou sous-groupe du système périodique des éléments, sous forme d'oxyde ou de sel, ou sous forme élémentaire et calcination à des températures de 300 à 130°°C et sous des pressions de 0,1 à 200 bars.

2. Procédé de préparation de catalyseurs, caractérisé en ce que l'on réunit des oxydes de la formule M-Al₂O₄, dans laquelle M représente un élément des premier, septième et huitième sous-groupes du système périodique des éléments, avec de l'étain, du plomb, un élément du second groupe principal ou sous-groupe du système périodique des éléments, sous forme d'oxyde ou de sel, ou sous forme élémentaire et on calcine à des températures de 300 à 1300°C et sous des pressions de 0,1 à 200 bars.

3. Procédé de préparation de catalyseurs suivant la revendication 2, caractérisé en ce que le substituant M dans les oxydes M-Al₂O₄ représente un élément des premier, septième et huitième sous-groupes du système périodique des éléments.

4. Procédé de préparation de catalyseurs suivant la revendication 2, caractérisé en ce que l'on utilise le zinc, le magnésium, le calcium, le strontium ou le baryum, sous forme d'oxyde ou de sel, ou sous forme élémentaire.

5. Procédé de préparation de catalyseurs suivant la revendication 2, caractérisé en ce que les composés des premier, septième et huitième sous-groupes du système périodique des éléments sont des oxydes de manganèse, de fer, de cobalt, de nickel ou de cuivre.
